# EUROPÄISCHE PATENTSCHRIFT

(11) **EP 1 330 262 B1**
(45) Veröffentlichungstag und Bekanntmachung des Hinweises auf die Patenterteilung: **18.05.2005**
(21) Anmeldenummer: 01992585.8
(22) Anmeldetag: 02.11.2001
(51) Int. Cl.: A61K 38/48, A61K 38/43, A61K 38/46, A61K 38/47, A61K 38/54, A61K 35/68, A61P 1/14

(54) **VERWENDUNG VON AUS CILIATEN GEWONNENEN ENZYMEN ALS VERDAUUNGSFÖRDERNDE ARZNEIMITTEL**
USE OF ENZYMES OBTAINED FROM CILIATES AS MEDICAMENTS FOR PROMOTING DIGESTION
UTILISATION D'ENZYMES OBTENUES A PARTIR DE CILIES COMME MEDICAMENTS FAVORISANT LA DIGESTION

(30) Priorität: 02.11.2000 DE 10054239
(43) Veröffentlichungstag der Anmeldung: 30.07.2003
(73) Patentinhaber: Cilian AG, 48149 Münster (DE)
(72) Erfinder: HARTMANN, Marcus, 48159 Münster (DE)
(74) Vertreter: Meyers, Hans-Wilhelm, Dr.Dipl.-Chem.
(86) Internationale Anmeldenummer: PCT/EP2001/012740
(87) Internationale Veröffentlichungsnummer: WO 2002/036156

(56) Entgegenhaltungen:
- US-A- 4 695 457
- ARAI-H ET AL.: "Properties of acid phospholipases in lysosomes and extracellular medium of Tetrahymena pyriformis" JOURNAL OF BIOCHEMISTRY, Bd. 99, 1986, Seiten 125-133, XP001052984
- FOK A K ET AL: "LYSOSOMAL ENZYMES OF PARAMECIUM CAUDATUM AND PARAMECIUM TETRAURELIA" EXPERIMENTAL CELL RESEARCH, SAN DIEGO, CA, US, Bd. 139, Nr. 1, 1. Mai 1982 (1982-05-01), Seiten 159-169, XP002043164 ISSN: 0014-4827

## Beschreibung

Die Erfindung betrifft ein Arzneimittel enthaltend Enzyme aus Ciliaten zur Behandlung von Verdauungsstörungen.

Verdauungsstörungen spielen in der allgemeinmedizinischen und internistischen Praxis eine immer größere Rolle. Diese Verdauungsstörungen sind in vielen Fällen Folge eines mehr oder minder ausgeprägten Mangels an sogenahnten Pankreasenzymen. Diese Enzyme werden im gesunden Zustand im Pankreas (Bauchspeicheldrüse) von hochspezialisierten Zellen, den sogenannten Azinuszellen synthetisiert und durch Exozytose über Speicheldrüsen und den Hauptausführgang (Ductus pancreaticus) in das Duodenum sekretiert. Die tägliche Menge an Pankreassekret beträgt ca. 2 Liter. Neben der fettverdauenden Lipase befinden sich im Pankreassekret auch Enzyme für die Verdauung von Proteinen (Trypsin, Chymotrypsin und Carboxypeptidasen) und Kohlenhydraten (α-Amylase). Dabei ist Sekretion von Pankreasenzymen durch endogene Regelmechanismen mittels von Hormonen, wie Gastrin, Sekretin und Pankreozymin genau geregelt. Dieses Regelwerk kann durch eine Vielzahl von Ursachen gestört werden, so dass es zur Drosselung der Pankreasenzymsekretion oder zum völligen Erliegen der exokrinen Pankreasfunktion kommt. Dies hat wiederum zur Folge, daß der Nahrungsbrei im Dünndarm nicht verdaut wird und eine Verdauungstörung auftritt. Diese auch als exokrine Pankreasinsuffizienz bezeichnete Erkrankung des Verdauungstraktes kann verschiedene Ursachen haben. Neben medikamentös bedingten Dyspepsien, chronischer atrophischer Gastritis und chronischer, häufig durch Alkoholgenuss verursachter, Pankreatitis sind operativ bedingte Störungen (z. B. Billroth I und II, Vagotomie, Pankreasresektion) und zystische Fibrose atiologische Faktoren der Pankreasinsuffizienz. In jedem Falle haben chronische Verdauungsstörungen eine erhebliche sozialmedizinische und damit volkswirtschaftliche Bedeutung, denn die Patienten sind häufig durch die Symptomatik eingeschränkt arbeitsfähig oder arbeitsunfähig und haben eine verkürzte Lebenserwartung.

Pankreatogene Verdauungsstörungen verursachen bei den Patienten eine Vielzahl von Beschwerden, wie Durchfälle, Massenstühle, Völlegefühl, Oberbauchbeschwerden, Gewichtsabnahme u.a..

Unabhängig von den Ursachen und der Ausprägung von pankreatogenen Verdauungsstörungen, bzw. einer Pankreasinsuffizienz ist zur Behebung der Verdauungsstörungen immer eine Substitutionstherapie mit Enzymen notwendig. Dies bedeutet, dass von außen die fehlenden Enzyme, überwiegend Lipase, Protease und Amylase, aber auch andere Enzyme zugeführt werden müssen. Bei der Therapie werden die Enzyme vom Patienten oral meist zur Mitte der Mahlzeit eingenommen und gelangen über den Magen in den Dünndarm, wo sie die Verdauung des Nahrungsbreis (Chymus) durchführen und somit die Funktion der fehlenden, körpereigenen Pankreasenzyme übernehmen. Dabei müssen Präparate zum Einsatz kommen, die über eine ausreichende Enzymmenge verfügen. Zudem müssen die Enzyme magensäureresistent, von geringer Partikelgröße und im Verdauungstrakt vollständig bioverfügbar sein.

Zur Behandlung von Verdauungsstörungen, die auf dem Fehlen von Pankreasenzymen, insbesondere dem Leitenzym Lipase und der Protease beruhen, sind bereits eine Vielzahl von Enzympräparaten auf dem Markt. Diese basieren zum Teil auf Pankreasenzymen aus Schweinen, wie z.B. die Präparate Combizym®, Festal®, Pankreon®, Kreon®, Panzytrat®, Meteozym® oder Enyzm-Lefax N@ oder, wie Citrapepsin®, auf Magen-Enzymen. Zum Teil enthalten die Präparate aber auch Enzyme aus Schimmelpilz-Extrakten, wie z.B. Combizym® und Festal®. Desweiteren wird der Einsatz von Enzymen aus Fischen oder anderen Meerestieren allgemein beschrieben (FR No. 1015566), sowie Kompositionen von Enzymen aus dem Magen-Darm-Trakt von Krill (Krebsarten der Klasse Euphausiaceae) und Capelin-Fischen (US4695457). Präparate, die Pankreasenzyme enthalten, werden meist aus den Schlachtabfällen, bzw. aus Bauchspeicheldrüsen von Schweinen gewonnen. Endprodukt des Herstellungsprozesses ist Pankreatin. Pankreatin ist ein Homogenat aus den Zellen der Pankreasgewebe (in der Regel vom Schwein). Es enthält durch das Aufbrechen einer Vielzahl von Azinuszellen neben Pankreasenzymen eine Vielzahl von weiteren Enzymen und Proteinen, sowie weiteren hoch- und niedermolekularen Verbindungen. Die Zusammensetzung von Pankreatin erklärt sich aus seinen industriellem Herstellungsprozess. Zur Gewinnung von Pankreatin werden Bauchspeicheldrüsen von Schweinen nach dem Schlachten so schnell wie möglich tiefgefroren, gesammelt und mechanisch aufgebrochen. Zur Stabilisierung und Aktivierung der Enzyme werden verschiedene Additive zum Homogenat hinzugegeben. Anschließend erfolgt die Entfettung mit organischen Lösungsmitteln wie z.B. Aceton, die Entfernung von Fasersubstanzen, sowie die Entwässerung und Trocknung durch Lyophilisierung. Für die Herstellung bestimmter Darreichungsformen kann eine galenische Weiterverarbeitung, zu Mikropellets, Tabletten, Kapseln, Pasten, Kremen, Gelen, Ölen oder anderen Formulierungen erfolgen. Häufig wird Pankreatin mit verschiedenen Trägermaterialien und Puffersubstanzen vermischt. Des weiteren wird granuliertes Pankreatin zum Schutz vor dem niedrigen pH-Wert des menschlichen Magensaftes mit säurestabilen Filmen oder Lacken überzogen. Die beiden letztgenannten Verarbeitungsschritte sollen dabei sicherstellen, däss die säurelabilen Pankreasenzyme am Zielort, dem Duodenum (Dünndarm), ihre Verdauungsfunktion erfüllen können.

Neben der üblichen Herstellung von Pankreatin kann der Lipase-Gehalt durch eine gestaffelte Extraktion mit Chloroform, Butanol und Aceton im Endprodukt gesteigert werden. Ähnlich wie beim Pankreatin werden Enzymkompositionen aus der Krillart Euphausia suberba und aus den Gedärmen der Capelin-Fische (in deutscher Sprache Lodden genannt) gewonnen (siehe US-A-4695457). Auch hier wird zunächst ein Homogenat aus dem Gewebe hergestellt, dass dann über Zentrifugation, Extraktion mit Chloroform, Lyophilisierung und chromatographische Schritte weiter eingereinigt wird. Ziel der Verarbeitungsprozesse ist in jedem Falle eine möglichst hoher Gehalt an Pankreasenzymen, wie Lipase, Protease und a-Amylase. Des weiteren soll die Enzymkomposition zur Behandlung von Verdauungsstörungen möglichst magensäureresistent sein. Außerdem sollen die Enzyme im Verdauungstrakt u. insbesondere im Duodenum möglichst schnell freigesetzt werden und ihre physiologische Wirkung entfalten. Für die Vermeidung von Allergien sollte die Enzymkomposition möglichst frei von nicht wirksamen Proteinen sein, bzw. einen hohen Reinheitsgrad aufweisen. Außerdem sollte auf Grund von pharmaökonomischen Gesichtspunkten der Herstellungsprozess kostengünstig sein.

Beim Pankreatin erfolgt üblicherweise keine weitere Aufreinigung über chromatographische Methoden, so dass die gewünschten Enzyme weder angereinigt noch homogen vorliegen. Nachteil solcher Proteingemische aus Zellhomogenaten ist, dass sie eine Vielzahl von Proteinen aus intrazellulären Zellkompartimenten (Cytosol, Zellkern, Membranen der Organellen) der Schweinepankreaszellen enthalten. Diese haben keine oder keine gewünschte enzymatische Aktivität und senken somit die Wirkstoffmenge pro zugeführter Darreichungsform erheblich. Dasselbe gilt, wenn ankonzentrierte Zellhomogenate aus Krill oder dem Magendarmtrakt von Capelin-Fischen gewonnen werden.

Ein anderer Nachteil der Herstellung von Enzymen und Enzymkompositionen aus Schlachtabfällen (Pankreas, Magendarmtrakt von Fischen) und Krill ist die diskontinuierliche Prozessführung der Gewinnung. Üblicherweise werden die Organe (Bauchspeicheldrüsen) in verschieden Schritten zerkleinert und homogenisiert. Das Homogenat wird anschließend entfettet und getrocknet. Diese Schritte der Enzymgewinnung lassen sich nicht kontinuierlich betreiben, da stets mit einem erheblichen Feststoffanteil gearbeitet wird, der sich auf Grund von Extraktions- und Zentrifugationsschritten nicht kontinuierlich weiterverarbeiten lässt. Für die Gewinnung von Enzymen sind jedoch kontinuierliche oder teilweise kontinuierliche Produktionsverfahren optimal, da die Raumzeitausbeuten in solchen Prozessen höher und die Kosten damit niedriger sind. Die kontinuierliche Gewinnung von Enzymen erfordert jedoch, dass diese extrazellulär in gelöster Form vorliegen und nicht erst durch Zerkleinerung bzw. Homogenisierung von Zellen freigesetzt werden müssen.

Für die kontinuierliche Gewinnung von Enzymen eignet sich der Pankreatin-Herstellungsprozess daher nicht

Ein weiterer Nachteil der Enzyme aus dem Pankreas ist ihre Säurelabilität. Pankreasenzyme sind neutrale oder alkalische Hydrolasen. Dies bedeutet, dass sie zum einen ihr Aktivitätsmaximum zwischen pH 7 und pH 8 haben und unter sauren Bedingungen eine stark verminderte Aktivität besitzen. Zum anderen inaktivieren niedrige pH-Werte ihre katalytische Funktion durch reversible oder irreversible Denaturierung. Aus diesem Grund müssen Enzyme, die aus Pankreas gewonnen werden (Pankreatin) durch spezielle Verfahren der Verkapselung oder Zugabe von Puffersubstanzen vor dem niedrigen pH-Wert der Magensäure bei der Magenpassage geschützt werden. Solche Verfahren erhöhen ebenfalls die Kosten von Arzneimitteln, die auf der Wirkung von Pankreatin beruhen.

Nachteilig für den Einsatz von Pankreas-Enzymen ist zudem für bestimmte Patientengruppen mit Verdauungsstörungen die Herkunft von Pankreatin. Üblicherweise werden die Bauchspeicheldrüsen von Schweinen verwendet, was für Patienten jüdischer oder moslemischer Religionszugehörigkeit auf Grund von religiösen Vorschriften nicht tolerierbar ist.

Schließlich können Pankreasenzyme aus Schweinen nicht bei Patienten mit Verdauungsstörungen eingesetzt werden, die eine Schweine-Eiweißallergie haben. Schweine gelten zudem als natürliches Reservoir von humanpathogenen Grippe-Viren, so dass eine Kontamination von Pankreatin mit solchen Viren nicht ausgeschlossen werden kann.

Aufgabe der Erfindung ist es daher, Enzyme bzw. Enzymkompositionen für Arzneimittel zur Verfügung zu stellen, die:
1) extrazellulär vorliegen und ohne Homogenisierung (Zerstörung) von Geweben oder Zellen aus zellfreiem Überstand gewonnen und aufgereinigt werden können.
2) sich in einem biotechnologischen Prozess eines ungefährlichen, nicht gentechnisch veränderten Mikroorganismus kontinuierlich gewinnen lassen.
3) saure Hydrolasen sind, also ihr Aktivitätsmaximum bei einem sauren pH-Wert haben und säurestabiler als Enzyme aus Pankreas sind.
4) nicht aus Geweben oder Organen des Schweins stammen.

Diese Aufgabe wird durch ein Arzneimittel enthaltend Enzyme, ausgewählt aus der Gruppe bestehend aus Hydrolasen, Lipasen, Proteasen, Amylasen, Glykosidasen, Phospholipasen, Phosphodiesterasen, Phosphatasen, gelöst, die aus Ciliaten gewonnen werden.

Gegenstand der Erfindung sind insbesondere Arzneimittel, die zur Förderung der Verdauung und zur Behandlung von Verdauungsstörungen eingesetzt werden.

Vorzugsweise enthalten die erfindungsgemäßen Arzneimittel Enzyme, die zum Verdau der in Nahrungsmitteln enthaltenden Makromoleküle, wie Proteinen, Nukleinsäuren und Kohlenhydraten und sowie sonstiger Bestandteile von Nahrungsmitteln, wie z.B. von Fetten oder Phospholipiden im Magen-Darm-Trakt von Menschen oder Tieren eingesetzt werden.

Dem Fachmann ist klar, dass erfindungsgemäß auch Enzyme aus Ciliaten zur Verdauungsförderung oder zur Behandlung von Verdauungsstörungen eingesetzt werden können, die nicht zu den bisher verwendeten Lipasen, Proteasen oder Amylasen gehören, da auch andere Enzyme durch die katalytische Spaltung von Bestandteilen der Nahrung die Verdauung im Magen-Darm-Trakt fördern können.

In einer Ausführungsform der Erfindung weisen die Enzyme ein pH-Optimum bei pH 4 - 6 auf.

Die erfindungsgemäßen Arzneimittel enthalten vorzugsweise Enzyme, die aus Ciliaten der Gattung Tetrahymena, Colpidium, Paramecium stammen.

Vorzugsweise sind in den erfindungsgemäßen Arzneimittel pharmazeutisch unbedenkliche Hilfs- und Trägerstoffe enthalten.

Die erfindungsgemäßen Arzneimittel werden insbesondere in Form von Tabletten, Mikropellets, Ölen, Säften, Gelen, Suppositorien, Kapseln, Dragees eingesetzt.

Gegenstand der Erfindung ist auch die Verwendung von Enzymen aus Ciliaten, ausgewählt aus der Gruppe bestehend aus Hydrolasen, Lipasen, Proteasen, Amylasen, Glykosidasen, Phospholipasen, Phosphodiesterasen, und/oder Phosphatasen zur Herstellung eines Arzneimittels zur Behandlung von Verdauungsstörung, insbesondere von durch medikamentös bedingter Dyspepsie, chronischer atropischer Gastritis, chronischer Pankreatitis, akuter Pankreatitis, einer operativ bedingten Verdauungsstörung (Maldigestion) oder die durch eine zystische Fibrose verursacht worden ist.

Die Enzyme, die Protozoen der Ordnung der Ciliaten produzieren und die in den erfindungsgemäßen Arzneimitteln eingesetzt werden, eignen sich sehr für die Behandlung von Verdauungsstörungen. Außerdem besitzen Enzyme und Enzymkompositionen aus Ciliaten, die in den erfindungsgemäßen Arzneimitteln enthalten sind, sowie ihre Herstellung nicht die Nachteile von den oben genannten Pankreasenzymen oder Enzymen aus Capelin-Fischen oder Krill.

Enzyme werden von Ciliaten in das sie umgebene Kulturmedium abgegeben. Tabelle 1 zeigt beispielsweise Enzymaktivitäten von verschiedenen Enzymen im Kulturmedium von Ciliaten. Die in Tabelle 1 dargestellten Enzymaktivitäten wurden mit den üblichen, in der Literatur beschriebenen Methoden bestimmt. Für die Messung der Lipase wurde ein Azo-Casein-Test verwendet (Muricane, 1986). Für die Bestimmung der Lipase und der Amylase wurden in Anlehnung an die Methodenvorschriften der FIP für fungale Amylase und mikrobielle Lipase durchgeführt (Demeester et al in "Pharmaceutical Enzymes", Lauwers, A. und Scharpé, S. [Hrsg.], Marcel Dekker, New York, 1997, pp. 372-382). Für die Bestimmung der sauren Phosphatase und der β-Hexosaminidase wurde ein p-Nitrophenylphosphat- bzw ein *p*-Nitrophenyl-*N*-acetyl-β-D-Glucosamin-Substrat eingesetzt, wie sie von Kiy et al. (1996) beschrieben wurden. Die Phospholipase A₁-Aktivität wurde mit einen radiometrischen Enzymtest (Hartmann et al., 2000) bestimmt.

**Tab. 1**

| Enzymaktivität im extrazellulären Kulturmedium (U/L) nach 72 h Kultivierung auf Magermilchmedium im 2-L-Fermenter | | | | | | |
|---|---|---|---|---|---|---|
| Ciliat | Protease | Lipase | α-Amylase | β-Hexosaminidase | Phospholipase A₁ | saure Phosphatase |
| *Tetrahymena* | 800 U/L | 164 U/L | 20 U/L | 500 U/L | 10 U/L | 1000 U/L |
| *Colpidium* | 12 U/L | - | - | 40 U/L | 1,5 U/L | 80 U/L |

Die Ciliaten, die Enzyme in das Kulturmedium abgeben, lassen sich kostengünstig auf preiswerten Fermentationsmedien in hoher Zelldichte und kontinuierlich fermentieren. Dabei lassen sich die Enzyme aus dem Fermenter über ein Perfusionsmodul (Mikrofilter) zellfrei abfiltrieren und so kontinuierlich aus dem Fermentationsmedium entfernen. Der Fermentationsprozess lässt über längere Zeiträume durch ständige Zuführung von kostengünstigen Nährmedien (Bestandteile: Magermilch-Medium und Hefeextrakt) aufrechterhalten.

### Beispiel 1

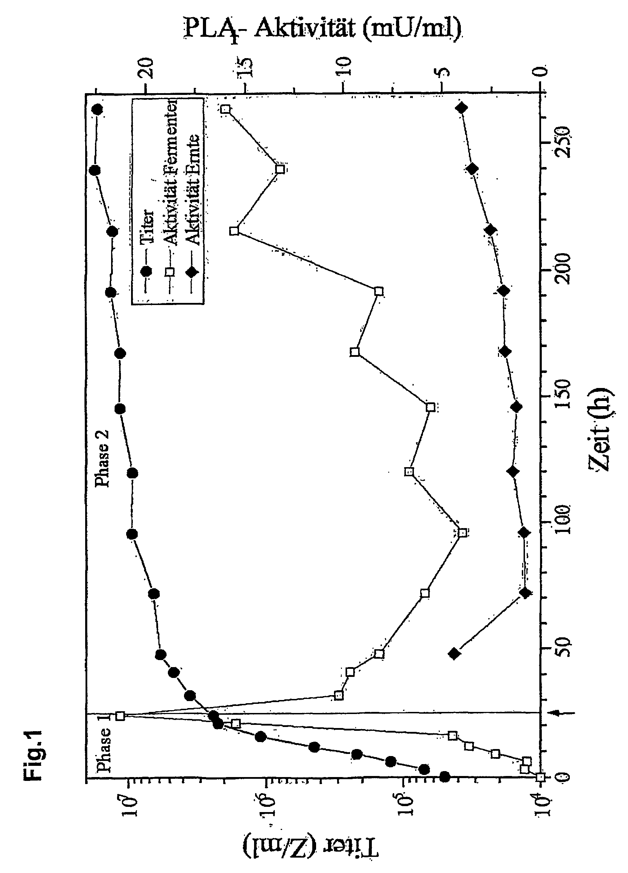

Fig. 1 zeigt die Sekretionskinetik eines Ciliaten über einen 14tägigen Fermentationszeitraum dargestellt. Für die kontinuierliche Fermentation mit Perfusionsmodul wurde dabei wie folgt verfahren:

Das Bioreaktorsystem basiert auf einem Prozessor-gesteuerten 2-L-Fermenter (BIOSTAT MD, BRAUN DIESSEL BIOTECH, Melsungen, Deutschland) mit einer digitalen DCU-Kontrolleinheit und einer Pumpeneinheit. Die Ernte des zellfreien Überstandes erfolgte über ein Perfusionsmodul, als Rührer wurde ein Paddelimpeller verwendet. Es wurde dabei eine Silikonölkonzentratlon von 1 ml/l verwendet. Die Umdrehungszahl des Rührers wurde zum Schutz vor Zellschäden auf 800 upm begrenzt. Die Konzentration des gelösten Sauerstoffes wurde mit Hilfe einer Kaskadenregelung konstant bei 60 % gehalten. Dabei wurde die Belüftungsrate als Regelgröße erster Priorität und die Rührerdrehzahl als Folgeregelung zweiter Priorität gewählt. Die Messung der Sauerstoffkonzentration erfolgte mit Hilfe einer amperemetrischen O₂-Elektrode (INGOLD MESSTECHNIK, Steinbach). Die Temperatur im Fermenter wurde über das Doppelwandgefäß und einen Thermostaten auf 30°C gehalten, die pH-Regulierung auf pH 7 erfolgte während der kontinuierlichen Fermentationsphase über eine DCU-gesteuerte Korrekturmittelpumpe mittels 4 M Essigsäure. In der kontinuierlichen Fermentationsphase wurde dem Fermenter Magermilchmedium zugeführt und das verbrauchte, enzymhaltige Medium aus dem Fermentationsprozess entfernt. Durch den Einsatz einer Pumpe, die über eine Leitfähigkeitssonde gesteuert wurde, konnte das Arbeitsvolumen konstant bei zwei Litern gehalten werden. Der zellfreie Überstand wurde über ein Perfusionsmodul mit einer Membranporengröße von ca. 0,3 µm partikelfrei geerntet. Das Perfusionsmodul bestand aus einer auf ein Haltegestell aufgerollten S6/2-Polypropylenkapillare (ENKA, Wuppertal) mit 2 bzw. 1 ml Außen- bzw. Innendurchmesser und einer Länge von 2,8 m. Der Austausch des Mediumvolumens pro Zeiteinheit wurde als Perfusionsrate definiert. Der Zulauf des Magermilchmediums konnte über die Drehzahl einer Schlauchpumpe so geregelt werden, dass sich eine Perfusionsrate von einem Fermentervolumen (zwei Liter) pro Tag einstellte. Vor dem Autoklavieren wurde der Fermenter mit der Schaumfalle vollständig montiert und mit 1,8 Liter Magermilchmedium ohne Glukose beschickt. Nach dem Autoklavieren wurden über den Zulauf 200 ml 10 %ige Glukoselösung zugepumpt. Der Anschluss der Medium- und der Ernteflasche erfolgte über Schnellkupplungen im Sterilkabinett. Die Animpfkultur wurde im Sterilkabinett in einen 500 ml Erlenmeyerkolben mit Bodenauslauf umgefüllt und über einen Schlauch und einen separaten Animpfstutzen in den Bioreaktor gepumpt.

Die Ciliaten bleiben bei der Fermentation unbeschädigt, so dass das Kulturmedium nur die von den Ciliaten ausgeschleusten Proteine und keine intrazellulären Bestandteile enthält. Aus diesem Grund lassen sich die Enzyme aus dem Fermentation- bzw. Kulturmedium mit wenigen chromatographischen Aufreinigungsschritten zu hoher Reinheit bringen.

### Beispiel 2

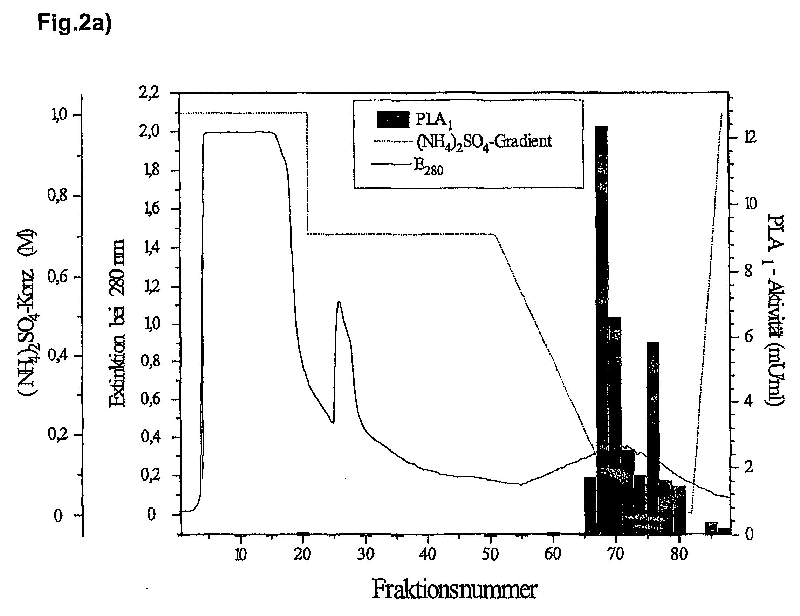

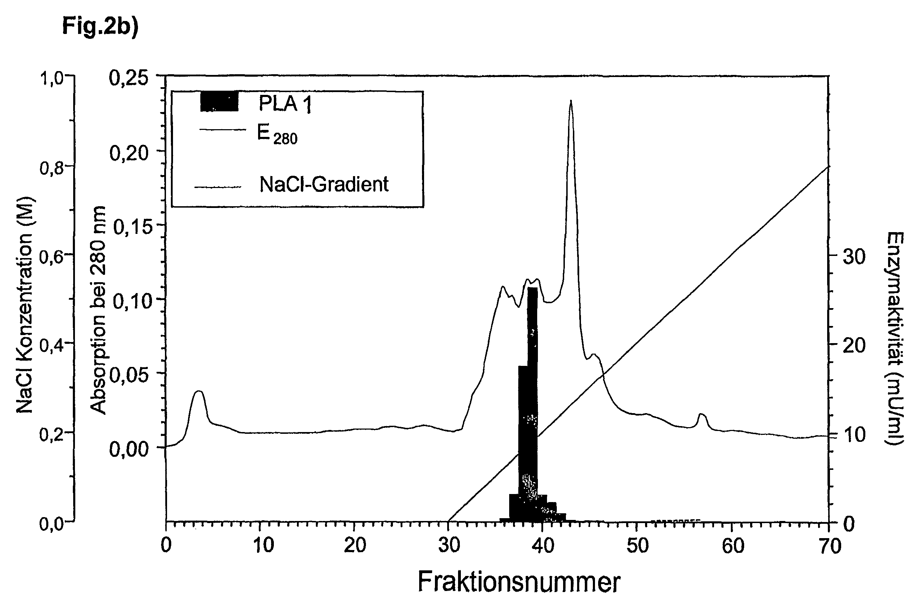

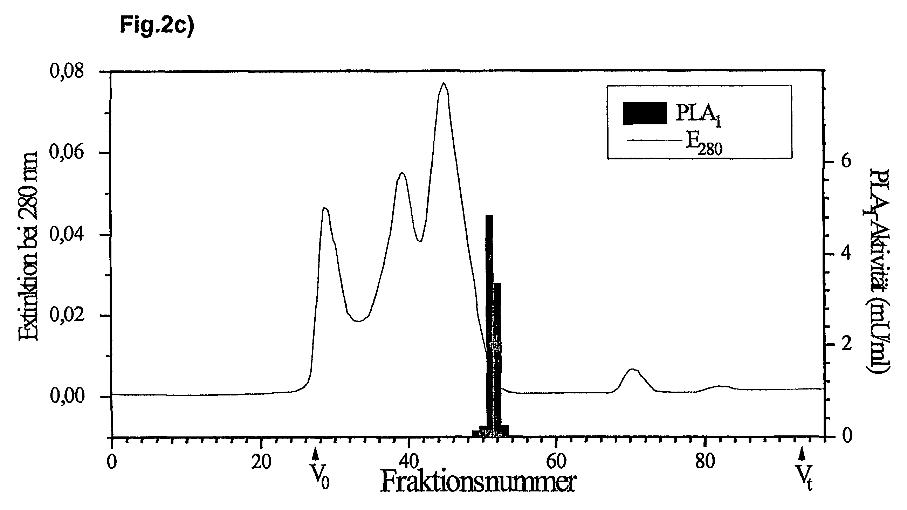

Fig. 2 sind beispielhaft säulenchromatographische Schritte zur Aufreinigung einer Phospholipase aus dem Kulturmedium vom Ciliaten *Tetrahymena* dargestellt. Dargestellt sind hierbei die Elutionsprofile dreier aufeinanderfolgender, säulenchromatographischer Schritte. Im einzelnen sind dabei in Fig. 2a als Schritt 1 das Elutionsprofil einer hydrophoben Interaktionschromatographie, in Fig. 2b als Schritt 2 das Elutionsprofil einer Anionenaustauscher-Chromatographie und in Fig. 2c als Schritt 3 das Elutionsprofil einer Größenausschlusschromatographie Für die folgenden Chromatographien wurden die aktiven Fraktionen der jeweils vorherigen verwendet. Das Enzym konnte so nach dem letzten Schritt zu fast vollständigen Homogenität aufgereinigt werden (keine Fremdaktivitäten, geringer Fremdproteinanteil). Vergleichbar mit diesem Aufreinigungschema lassen sich auch andere Enzyme aus Ciliaten, wie Protease, Lipase, Amylase oder β-Hexosaminidase aufreinigen.

Ciliaten sind, bis auf einen fakultativ pathogenen Vertreter (Balantidium coli) freilebende (nicht parasitische) apathogene Mikroorganismen. So ist beispielsweise für den Ciliaten Tetrahymena der GRAS-Status ("general recognized as save") in der Literatur belegt (Tiedtke, 1994). Es gilt zudem als sicher, dass Ciliaten keine Endoparasiten beherbergen, die auf andere Organismen übertragen werden können. Bei den für die Biotechnologie wichtigen Ciliaten-Arten, wie Tetrahymena oder Colpidium existieren zudem keine Viren oder sonstigen Endoparasiten. Eine Kontamination der Enzymkomposition mit toxischen oder pyrogenen Verunreinigungen kann daher ausgeschlossen werden.

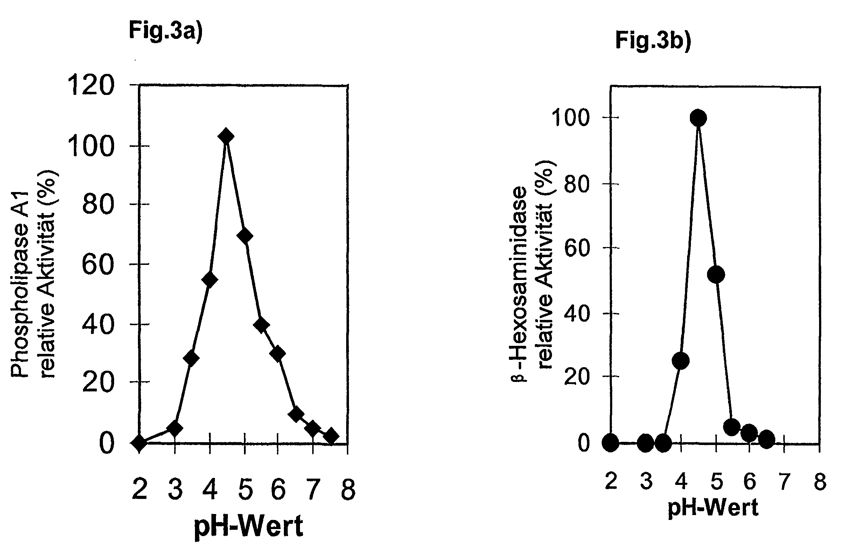

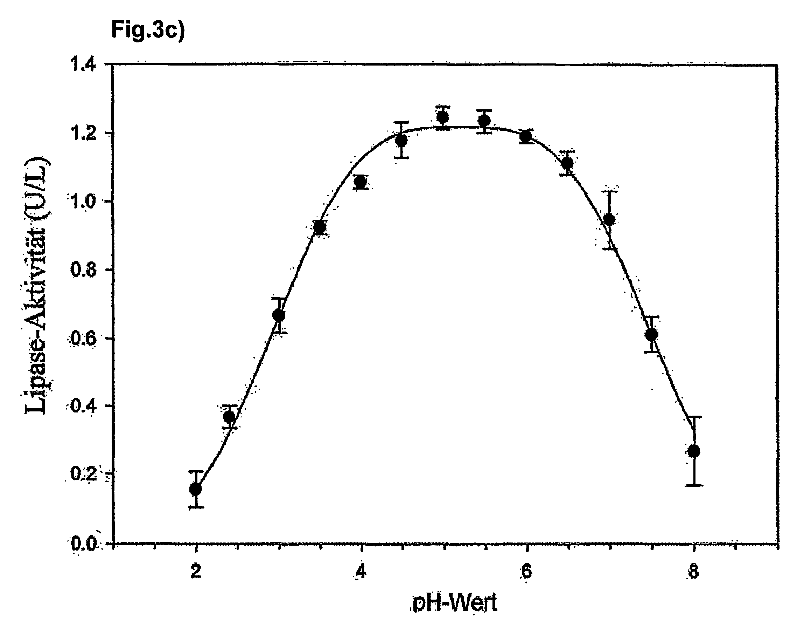

Fig.3 zeigt die Darstellung der relativen Enzymaktivitäten von 3 Enzymen aus dem Kulturmedium vom Ciliaten *Tetrahymena* bei verschiedenen pH-Werten (a - Phospholipase A₁, b - Triacylglycerollipase, c - β-Hexosaminidase).

Die Enzyme aus Ciliaten haben als saure Hydrolasen ein saures pH-Optimum. In Fig. 3 sind die Enzymaktivitäten von 3 Enzymen aus dem Kulturmedium vom Ciliaten *Tetrahymena* bei verschiedenen pH-Werten. Im einzelnen sind die relativen Enzymaktivitäten der Phospholipase A₁ (Fig. 3a) und der β-Hexosaminidase (Fig. 3b), sowie die absolute Enzymaktivität der Upase (Fig. 3c) dargestellt.

Hieran wird deutlich, dass das pH-Optimum für Enzyme aus Ciliaten zwischen pH 4.1 und 6,5 liegt. Die Protease aus Ciliaten zeigt eine hohe Enzymaktivität sogar noch bei einem pH-Wert von 3. In der folgenden Tabelle 2 ist die Aktivität der Ciliaten-Protease aus dem zellfreien Überstand (Kulturmedium) in Abhängigkeit vom pH-Wert dargestellt. Die Enzymaktivitäten wurden dabei, wie bereits oben beschrieben, in Anlehnung an die Methodenvorschriften der FIP für fungale Amylase und mikrobielle Lipase durchgeführt.

**Tab. 2**

| pH-Wert | 3 | 4 | 5 | 6 |
|---|---|---|---|---|
| Protease-Aktivität (Units/Liter) | 2266 ± 19 % | 1854 ± 12 % | 1483,2 ± 11 % | 11948 ± 25 % |

Aus diesem Grund sind Ciliaten-Enzyme auch säurestabiler als Pankreasenzyme. Infolge dessen haben sie nach einer Magenpassage eine erheblich höhere Aktivität im Duodenum, als Pankreas-Enzyme.

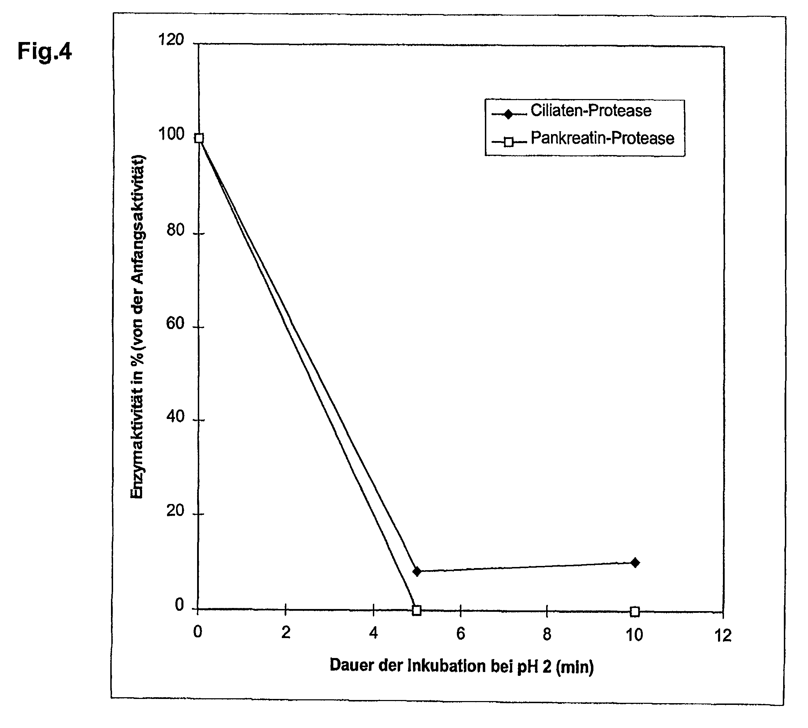

In Fig. 4 ist die Säurestabilität einer Protease aus dem Ciliaten *Tetrahymena* gegen über Pankreatin bei einer 10 minütigen Einwirkphase von einem typischen pH-Wert, wie er im Magensaft vorliegt (pH 1,5) dargestellt. Der niedrige pH-Wert wurde durch das Einwirken eines hochmolaren, sauren Puffers ((1 M Glycin/HCL, pH 1,5) bei 37°C simuliert.

## Patentansprüche

1. Enzyme aus Ciliaten zur Verwendung als Arzneimittel, ausgewählt aus der Gruppe bestehend aus Hydrolasen, Lipasen, Proteasen, Amylasen, Glykosidasen, Phospholipasen, Phosphodiesterasen, Phosphatasen.

2. Enzyme aus Ciliaten nach Anspruch 1, **dadurch gekennzeichnet, dass** die Enzyme ein pH-Optimum bei pH 4 - 6 besitzen.

3. Enzyme aus Ciliaten nach einem der Ansprüche 1 und/oder 2, **dadurch gekennzeichnet, dass** die Enzyme aus Ciliaten der Gattung Tetrahymena, Colpidium, Paramecium, stammen.

4. Enzyme aus Ciliaten nach einem der Ansprüche 1 bis 3, **dadurch gekennzeichnet, dass** es weiterhin pharmazeutische Hilfs- und Trägerstoffe enthält.

5. Enzyme aus Ciliaten nach einem der Ansprüche 1 bis 4, **dadurch gekennzeichnet, dass** es in Form von Tabletten, Mikropellets, Ölen, Säften, Gelen, Suppositorien, Kapseln, Dragees vorliegt.

6. Verwendung von Enzymen aus Ciliaten, ausgewählt aus der Gruppe bestehend aus Hydrolasen, Lipasen, Proteasen, Amylasen, Glykosidasen, Phospholipasen, Phosphodiesterasen, und/oder Phosphatasen zur Herstellung eines Arzneimittels zur Behandlung von Verdauungsstörung.

7. Verwendung gemäß Anspruch 6, wobei die Verdauungsstörungen durch medikamentös bedingte Dyspepsie, chronische atropische Gastritis, chronische Pankreatitis, akute Pankreatitis, eine operativ bedingte Verdauungsstörung (Maldigestion) oder durch eine zystische Fibrose verursacht worden sind.

## Claims

1. Enzymes from ciliates for use as a medicament, selected from the group consisting of hydrolases, lipases, proteases, amylases, glycosidases, phospholipases, phosphodiesterases, phosphatases.

2. The enzymes from ciliates according to claim 1, **characterized in that** said enzymes have a pH optimum at pH 4-6.

3. The enzymes from ciliates according to any of claims 1 and/or 2, **characterized in that** said enzymes are derived from ciliates of the genera *Tetrahymena, Colpidium* and *Paramecium*.

4. The enzymes from ciliates according to any of claims 1 to 3, **characterized in that** said medicament further contains pharmaceutical auxiliary agents and carriers.

5. The enzymes from ciliates according to any of claims 1 to 4, **characterized in that** said medicament is in the form of tablets, micropellets, oils, juices, gels, suppositories, capsules, coated tablets.

6. Use of enzymes from ciliates selected from the group consisting of hydrolases, lipases, proteases, amylases, glycosidases, phospholipases, phosphodiesterases and/or phosphatases for the preparation of a medicament for treating digestive disorders.

7. The use according to claim 6, said digestive disorders being caused by dyspepsia caused by medicaments, chronic atrophic gastritis, chronic pancreatitis, acute pancreatitis, digestive disorder (maldigestion) caused by surgery, or by cystic fibrosis.

## Revendications

1. Enzymes obtenues à partir de ciliés destinées à être utilisées comme médicament, choisies dans le groupe constitué par les hydrolases, les lipases, les protéases, les amylases, les glycosidases, les phospholipases, les phosphodiestérases, les phosphatases.

2. Enzymes obtenues à partir de ciliés selon la revendication 1, **caractérisées en ce que** les enzymes possèdent un pH optimal de pH 4 à 6.

3. Enzymes obtenues à partir de ciliés selon l'une des revendications 1 et/ou 2, **caractérisées en ce que** les enzymes sont issues de ciliés des genres *Tetrahymena, Colpidium, Paramecium.*

4. Enzymes obtenues à partir de ciliés selon l'une des revendications 1 à 3, **caractérisées en ce qu'**elles contiennent en outre des substances auxiliaires et des véhicules acceptables en pharmacie.

5. Enzymes obtenues à partir de ciliés selon l'une des revendications 1 à 4, **caractérisées en ce qu'**elles se présentent sous forme de comprimés, de micropastilles, d'huiles, de jus, de gelées, de suppositoires, de gélules, de dragées.

6. Utilisation d'enzymes obtenues à partir de ciliés, choisies dans le groupe constitué par les hydrolases, les lipases, les protéases, les amylases, les glycosidases, les phospholipases, les phosphodiestérases, et/ou les phosphatases pour la fabrication d'un médicament destiné à traiter les troubles digestifs.

7. Utilisation selon la revendication 6, les troubles digestifs étant provoqués par une dyspepsie d'origine médicamenteuse, une gastrite atrophique chronique, une pancréatite chronique, une pancréatite aiguë, des troubles digestifs d'origine opératoire (maldigestion) ou par la mucoviscidose.
